# EUROPÄISCHE PATENTSCHRIFT

(11) **EP 1 054 700 B1**
(45) Veröffentlichungstag und Bekanntmachung des Hinweises auf die Patenterteilung: **07.09.2005**
(21) Anmeldenummer: 99908821.4
(22) Anmeldetag: 25.01.1999
(51) Int. Cl.: A61M 1/36, A61M 39/10

(54) **AUFNAHMEGEFÄSS FÜR KÖRPERFLÜSSIGKEITEN MIT EINEM ADAPTERSTOPFEN**
RECEPTACLE FOR BODY FLUIDS COMPRISING AN ADAPTER STOPPER
RECIPIENT POUR LIQUIDES ORGANIQUES, COMPORTANT UN BOUCHON ADAPTATEUR

(30) Priorität: 11.02.1998 DE 19805941; 24.07.1998 DE 19833435
(43) Veröffentlichungstag der Anmeldung: 29.11.2000
(73) Patentinhaber: Müller, Hans, 81547 München (DE)
(72) Erfinder: Müller, Hans, 81547 München (DE)
(74) Vertreter: Schaeberle, Steffen
(86) Internationale Anmeldenummer: PCT/EP1999/000461
(87) Internationale Veröffentlichungsnummer: WO 1999/040956

(56) Entgegenhaltungen:
- EP-A- 0 240 144
- EP-A- 0 787 503
- DE-A- 19 531 751
- US-A- 5 425 465

## Beschreibung

Die Erfindung betrifft ein Aufnahmegefäß für Körperflüssigkeiten mit einem Adapterstopfen zum Be- und Entladen mit Körperflüssigkeiten.

Zu medizinischen Therapieverfahren werden dem Patienten entnommene Körperflüssigkeiten mit UV-Licht bestrahlt. Vorrichtungen zu diesem Zweck kennt man aus der deutschen Patentschrift 195 31 751, wobei die entnommene Körperflüssigkeit in eine Quarzküvette überführt und anschließend mit UV-Licht bestrahlt wird. Zur Be- und Entladung wird die Körperflüssigkeit mit einer Spritze durch eine Be- und Entladeöffnung eines Adapterstopfens, der die Quarzküvette verschließt, gespritzt. Der hierbei in der Quarzküvette entstehende Überdruck kann durch eine Lüftungsöffnung am gegenüberliegenden Stopfen entweichen.

Dies ist jedoch nur gewährleistet, solange die Quarzküvette beim Einspritzen der Körperflüssigkeit in das Aufnahmegefäß nach oben gehalten wird, wobei dann die Körperflüssigkeit von unten eingespritzt wird. Bei unsachgemäßer Handhabung, wenn z. B. die Körperflüssigkeit waagrecht oder gar nach unten gerichtet in die Quarzküvette gespritzt wird, kann die erwünschte Entlüftung nicht mehr stattfinden. In diesem Fall kommt die Lüftungsöffnung bereits in Kontakt mit der Körperflüssigkeit, bevor das gesamte Volumen der Körperflüssigkeit in die Quarzküvette gespritzt wurde. Dies bedeutet, daß, noch bevor der Überdruck aus der Quarzküvette entwichen ist, geringe Mengen der Körperflüssigkeit in die Lüftungsöffnung eindringen. Diese Körperflüssigkeit wird beim Entweichen des restlichen Überdruckes durch die Lüftungsöffnung gepreßt und verschmutzt die Außenfläche des Stopfens bzw. der Quarzküvette. Durch das Einführen der außen verschmutzten Quarzküvette in das Bestrahlungsgerät wird auch dieses verschmutzt. Solche Verschmutzungen mit Körperflüssigkeiten stellen bevorzugte Keimreservoirs dar und erhöhen damit die Wahrscheinlichkeit eines Kontaminationsrisikos für nachfolgende Proben.

Dieses Risiko ist besonders erhöht, wenn beim Entnehmen der Körperflüssigkeit durch absaugen aus der Quarzküvette die in die Lüftungsöffnung gelangte Körperflüssigkeit mit abgesaugt wird. Da davon ausgegangen werden kann, daß die Außenbereiche nicht steril sind, wird hierbei die gesamte Probe kontaminiert.

Durch die EP-A-240 144 ist wohl ein Aufnahmegefäß für Medikamente bekannt, bei welchem im Verschlußstopfen eine Belüftungsöffnung vorgesehen ist, die jedoch mit einem einsteckbaren Verschlußstopfen verschließbar ist. Der Verschlußstopfen ist an der Belüftungsöffnung über einen angespritzten Verbindungsstreifen befestigt. Die Herstellung des Verschlußstopfens erfordert ein sehr aufwendiges Spritzwerkzeug, was unerwünscht ist.

Es ist daher Aufgabe der Erfindung, eine Vorrichtung zur sterilen Beladung und Entladung einer Quarzküvette zur Bestrahlung von Körperflüssigkeit mit einem Bestrahlungsgerät zur Verfügung zu stellen, bei welcher gewährleistet ist, daß bei unsachgemäßer Handhabung das Risiko einer Kontamination der Außenflächen des Aufnahmegefäßes bzw. der Bestrahlungsvorrichtung verringert wird, wobei aus Kostengründen sowie um eine möglichst universelle Verwendbarkeit zu gewährleisten, eine Lösung gesucht wird, die einen möglichst geringen apparativen Aufwand und geringe Herstellungskosten erfordert.

Ausgehend von dem eingangs erwähnten Aufnahmegefäß wird diese Aufgabe erfindungsgemäß dadurch gelöst, daß das aus einer Quarzküvette bestehende Aufnahmegefäß an der dem Adapterstopfen gegenüberliegenden Seite mit einem stopfenartigen Verschlußelement verschlossen ist, und daß die Lüftungsbohrung am Steckstopfen des Adapterstopfens in einem durch das Darüberschieben der Quarzküvette verschließbaren Bereich angebracht ist.

Der Verschluß des erfindungsgemäßen Aufnahmegefäßes erfolgt durch ein Verschlußelement, z. B. durch einen an den Gefäßinnenflächen dicht schließenden Stopfen oder eine an den Gefäßaußenflächen dicht abschließende Aufsatzkappe. Durch die am Steckstopfen des Adapterstopfens angebrachte Lüftungsbohrung ist in einfacher Weise ein Gasaustausch und damit ein Druckausgleich beim Be- und Entladen des Aufnahmegefäßes möglich.

Bei dieser Ausgestaltung ist es vorteilhaft, den Adapterstopfen zum Beladen bis zum Anschlag in die Quarzküvette einzustecken. In dieser Anordnung ist die Lüftungsöffnung durch die Quarküvette abgedichtet, so daß es sich um ein dicht abgeschlossenes System handelt. Der beim Beladen entstehende Überdruck kann durch leichtes Schwenken der Küvette über den Nadelkonus der Spritze entweichen. Aus einem derart abgeschlossenen System kann während der Bestrahlung oder Handhabung der Küvette keine Körperflüssigkeit entweichen, so daß eine Kontamination der Bestrahlungsvorrichtung ausgeschlossen ist. Zum Entladen der Küvette wird dann der Adapterstopfen so weit aus der Küvette gezogen, daß die Lüftungsöffnung am oberen Rand des Steckstopfens freiliegt. Um ein zu weites Herausziehen des Steckstopfens und damit eine instabile Handhabung zu vermeiden, ist es vorteilhaft, die Lüftungsbohrung nahe dem oberen Rand anzubringen. Durch diese Lüftungsbohrung kann nun - im herausgezogenen Zustand - beim Absaugen der Körperflüssigkeit Luft in die Quarzküvette nachströmen und so den entstehenden Unterdruck ausgleichen.

Eine weitere erfindungsgemäße Lösung der Aufgabe sieht vor, daß die Lüftungsbohrung derzeit bekannter Verschlußelemente mit einer Abschlußkappe verschließbar ist. Diese Abschlußkappe rastet über einen Rastring in eine seitliche Vertiefung des Verschlußelementes ein, so daß gewährleistet ist, daß die Abschlußkappe nicht beim Herausziehen aus der Bestrahlungsvorrichtung vom Verschlußelement herunterrutschen kann. Eine sterile Abschlußkappe verhindert somit den Kontakt zwischen eventuell ausgetretener Körperflüssigkeit und der Bestrahlungsvorrichtung. Wie schon oben erwähnt, verringert sich dadurch das Kontaminationsrisiko für die Bestrahlungsvorrichtung und damit auch, trotz unsachgemäßer Handhabung, für weitere Proben.

Eine besonders vorteilhafte Ausführungsform der Erfindung sieht vor, daß die Durchbohrung des Verschlußelements mit einer gasdurchlässigen und flüssigkeitsundurchlässigen Mikromembran versehen ist. Eine solche Mikromembran erlaubt den Gasaustritt durch Mikroporen, während ein Flüssigkeitsdurchtritt aufgrund der Porengröße nicht möglich ist. In einem Ausführungsbeispiel der vorliegenden Erfindung ist die Mikromembran an der Innenseite des Verschlußelementes angebracht. In diesem Fall sind vorzugsweise zwischen Verschlußelement und Membran Abstandshalter einzufügen, die z. B. am Rand der Innenseite des Verschlußelementes aus einem umlaufenden und vorstehenden Rand bestehen können. Diese Abstandshalter gewährleisten, daß die gesamte Membranoberfläche für einen Gasaustausch zur Verfügung steht.

In einer weiteren Ausführungsform der Erfindung ist diese Mikromembran als Gefäßöffnung umspannende Aufsteckkappe ausgebildet. Als besonders vorteilhaft bei diesen Ausführungsformen mit Mikromembranabschluß ist zu erwähnen, daß ein durch falsche Handhabung verursachtes Verstopfen der Mikroporen, wodurch ein Gasaustausch unterbunden würde, wegen der großen Anzahl von Mikroporen ausgeschlossen ist.

Im folgenden wird die Erfindung anhand einer Zeichnung von Ausführungsbeispielen näher erläutert. Es zeigen
- Fig. 1: einen Längsschnitt durch eine Bestrahlungsvorrichtung für die Behandlung von Körperflüssigkeiten;
- Fig. 2: einen Längsschnitt durch einen Adapterstopfen im verschlossenen Zustand, der eine Lüftungsbohrung am Steckstopfen hat;
- Fig. 3: einen Längsschnitt durch einen Adapterstopfen im belüfteten Zustand, der eine Lüftungsbohrung am Steckstopfen hat;
- Fig. 4: ein Verschlußelement mit seitlicher Entlüftungsbohrung;
- Fig. 5: einen Längsschnitt durch ein Verschlußelement ohne Lüftungsöffnung;
- Fig. 6: einen Längsschnitt durch ein Verschlußelement mit einer in eine Lüftungsöffnung eingesetzten Mikromembranabdichtung für ein Aufnahmegefäß für Körperflüssigkeiten;
- Fig. 7: einen Längsschnitt durch ein Verschlußelement mit integrierter Mikromembranabdichtung für ein Aufnahmegefäß für Körperflüssigkeiten;
- Fig. 8: einen Längsschnitt durch ein Verschlußelement mit aufgesetzter Abschluß kappe.

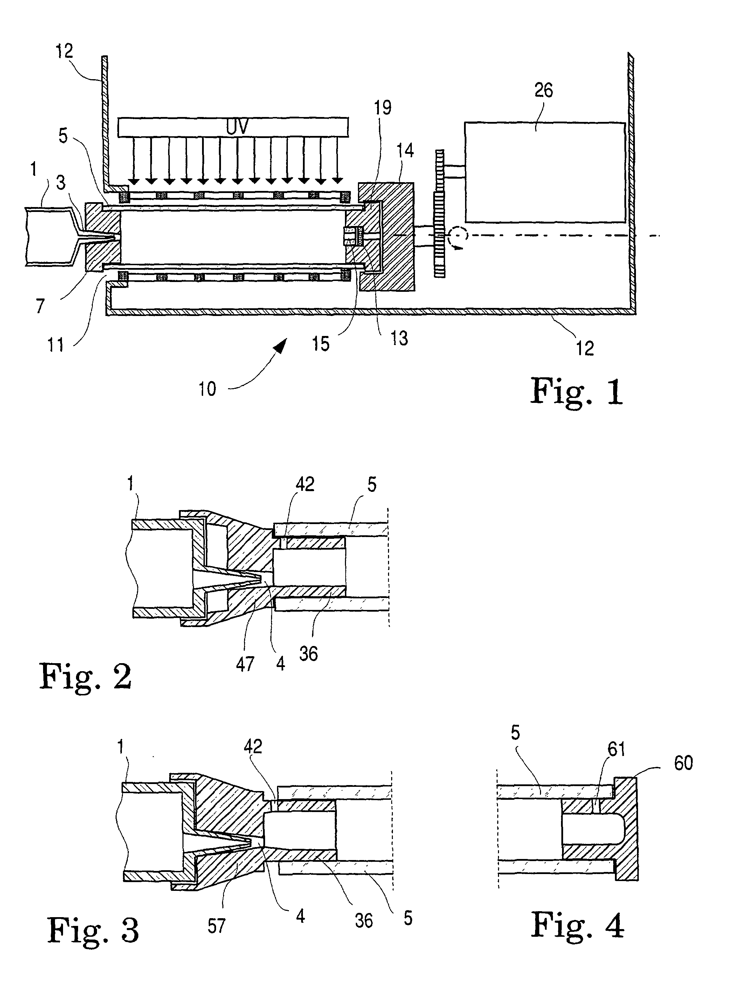

In der in Fig. 1 gezeigten Anordnung ist das Gehäuse 12 einer bekannten Bestrahlungsvorrichtung 10 im Längsschnitt zu sehen. Das Gehäuse 12 weist eine Aufnahmeöffnung 11 zum Einführen einer als Aufnahmegefäß für Körperflüssigkeiten ausgebildeten Quarzküvette 5 auf. Die Aufnahmeöffnung 11 führt ins Innere eines Führungsrohres, in welchem eine Halterung 14 an einer axialen Antriebswelle befestigt ist. Das in Einführungsrichtung vorne liegende Ende der Küvette 5 ist mit einem Verschlußelement 19 abgedichtet, welches in die Halterung 14 einschnappt, so daß die Drehbewegung des Antriebsmotors 26 auf die Quarzküvette übertragen wird. Das Verschlußelement 19 ist mit einer Durchbohrung 15 zur Be- und Entlüftung mit integriertem Bakterienfilter 13 versehen. Die Quarzküvette 5 kann mit einer Spritze 1, die zu behandelnde Körperflüssigkeit enthält, durch Einsetzen des Nadelkonus 3 in eine Bohrung eines Adapterstopfens 7 befüllt werden. Beim Einfüllen der Körperflüssigkeit in die Quarzküvette kommt es über die Bohrung 15 am Verschlußelement 19 zum erwünschten Druckausgleich. Nach Beendigung des Bestrahlungsvorgangs wird die Quarzküvette 5 aus der Halterung 14 ausgeklinkt und aus dem Gehäuse 12 entnommen. Anschließend kann z. B. der bestrahlte Inhalt mit der Spritze wiederum aus der Quarzküvette abgesaugt werden, um sie dem Patienten zurückzuapplizieren. Das Bakterienfilter 13 verhindert dabei eine Verkeimung des Quarzküvetteninhalts beim Zurückströmen von Luft in die sich leerende Quarzküvette 5.

In Fig. 2 und 3 sind zwei Positionen von Adapterstopfen 47 und 57, welche gemäß den sich unterscheidenden Darstellungen unterschiedlich gestaltet sein können, zum Verschluß des Aufnahmegefäßes 5 gezeigt. Diese Adapterstopfen weisen nahe dem oberen Rand des Steckstopfens 36 eine Lüftungsbohrung 42 auf. Während in Fig. 2 der geschlossene Zustand dargestellt ist, indem die Lüftungsbohrung 42 durch das Aufnahmegefäß 5 abgedichtet wird, zeigt Fig. 3 einen Zustand mit herausgezogenem Steckstopfen 36, bei dem die Lüftungsöffnung 42 freiliegt und somit ein Gasaustausch möglich ist.

Die Entlüftung kann auch gemäß Fig. 4 über eine seitliche Bohrung 61 im Steckstopfen des Verschlußelements 60 erfolgen, wobei die Handhabung entsprechend der bei der Entlüftung über den Adapterstopfen gemäß den Fig. 2 und 3 ist.

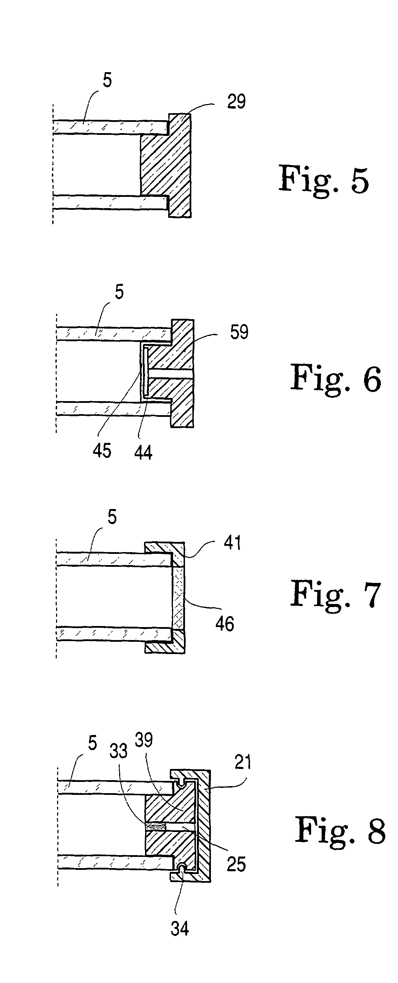

In Fig. 5 ist das hintere Ende eines Aufnahmegefäßes zur Bestrahlung von Körperflüssigkeiten dargestellt, welche in ein Bestrahlungsgerät 10 gemäß Fig. 1 einsetzbar ist. Während das in Einführungsrichtung vorne liegende Ende der Quarzküvette 5 mit einem stopfenförmigen Verschlußelement 29 verschlossen ist, kann das in Einführungsrichtung hinten liegende Ende der Quarzküvette mit einem Adapterstopfen gemäß Fig. 3 abgedichtet sein, wobei der Adapterstopfen mit einem Steckstopfen 36 in die Quarzküvette 5 ragt, und einem trichterförmigen Aufsteckkonus 4 zum Einsetzen der Spritze 1 umfaßt. In diesen Aufsteckkonus wird der Nadelkonus der Spritze 1 zum Be- und Entladen des Aufnahmegefäßes eingesteckt. Neben dem Aufsteckkonus 4 zum Be- und Entladen ist die Lüftungsbohrung 42 im Steckstopfen 36 zum Be- und Entlüften der Quarzküvette angebracht. Durch diese Lüftungsbohrung 2 entweicht Luft beim Beladen der Quarzküvette nach außen.

Die weiteren Figuren zeigen verschiedene Abwandlungen der Verschlußelemente des Aufnahmegefäßes zur Bestrahlung von Körperflüssigkeiten. Selbstredend sind beliebige Kombinationen von Adapterstopfen und Verschlußelementen 29, 39, 59, 60, 41 zum Abdichten des Aufnahmegefäßes möglich.

In Fig. 6 ist ein Verschlußelement 59 mit Abstandshaltern 44 gezeigt, welches von dem Küvetteninhalt durch eine Mikromembran 45 abgetrennt ist. Diese Abstandshalter 44 sind am äußeren Rand der Innenfläche des Verschlußelementes 59 angebracht. Die Fläche der Mikromembran, ausgehend von diesen Abstandshaltern 44 radial nach innen, dient dem Gasaustausch. Der Gasaustausch erfolgt hierbei durch Mikroporen, während ein Flüssigkeitsdurchtritt aufgrund der Porengröße nicht möglich ist. Nach einem Membrandurchtritt kann die Luft durch die Lüftungsöffnung des Verschlußelements 59 zirkulieren.

In Fig. 7 ist eine durch eine Abschlußklappe 41, in die eine Mikromembran 46 eingesetzt ist, abgedichtete Quarzküvette abgebildet. Durch den Einsatz der Mikromembran 46 ist ein Gasaustausch über die Gesamtfläche des Küvettendurchmessers jedoch kein Austreten von Flüssigkeiten möglich. Eine solche Mikromembran kann auch mit einer nicht dargestellten Ringkappe auf das Ende der Quarzküvette aufgezogen werden.

Die in Fig. 8 dargestellte Ausführungsform des Aufnahmegefäßes zur Bestrahlung von Körperflüssigkeiten unterscheidet sich auf der Seite des Adapterstopfens an der dem Adapterstopfen gegenüberliegenden Seite durch ein durchbohrtes Verschlußelement 39, welches mit einer Abschlußkappe 21 abgedichtet wird. Die Abschlußkappe 21 ist über einen Rastring 34 mit dem Verschlußelement 39 verbunden. Dieser Rastring 34 schnappt beim Aufsetzen der Abschlußkappe 21 ein und verhindert, daß die Abschlußkappe 21 beim Herausnehmen der Quarzküvette aus der Bestrahlungsvorrichtung herunterrutscht und in der Halterung des Bestrahlungsgerätes verbleibt. Falls gewünscht, kann bei entsprechender Ausgestaltung jedoch auch Luft durch die Lüftungsbohrung 25 des Verschlußelements 39 und weiter zwischen Verschlußelement und Abschlußkappe 21 entweichen. Auch in diesem Fall verhindert ein in die Lüftungsbohrung 25 integriertes Bakterienfilter 33 eine Verkeimung der Quarzküvette durch eventuell zurückströmende Luft.

## Patentansprüche

1. Aufnahmegefäß für Körperflüssigkeiten mit einem mit einer verschließbaren Lüftungsbohrung versehenen Adapterstopfen (47), der zum Be- und Entladen mit Körperflüssigkeiten mit einer Durchbohrung (4) zum Einsetzen eines Nadelkonus (3) einer Spritze (1) versehen ist und einen auf die Öffnung des Aufnahmegefäßes aufsteckbaren Steckstopfen (36) hat,
**dadurch gekennzeichnet,**
**daß** das aus einer Quarzküvette (5) bestehende Aufnahmegefäß an der dem Adapterstopfen (47; 57) gegenüberliegenden Seite mit einem stopfenartigen Verschlußelement (29; 21, 39; 31, 49; 45, 59; 41, 46; 60) verschlossen ist,
und **daß** die Lüftungsbohrung (42) am Steckstopfen (36) des Adapterstopfens (47) in einem durch das Darüberschieben der Quarzküvette (5) verschließbaren Bereich angebracht ist.

2. Aufnahmegefäß nach Anspruch 1,
**dadurch gekennzeichnet,**
**daß** eine Durchbohrung (25; 35) des stopfenartigen Verschlußelements (39; 49) mit einer Abschlußkappe (21; 31) verschließbar ist.

3. Aufnahmegefäß nach Anspruch 1,
**dadurch gekennzeichnet,**
**daß** das Verschlußelement (41; 59) mit einer gasdurchlässigen und flüssigkeitsundurchlässigen Membran (45; 46) versehen ist.

4. Aufnahmegefäß nach Anspruch 3,
**dadurch gekennzeichnet,**
**daß** die Membran (46) in bzw. mit einer Ringkappe (41) gehalten ist.

5. Aufnahmegefäß für Körperflüssigkeiten mit einem Adapterstopfen (47; 57), der zum Be- und Entladen mit Körperflüssigkeiten mit einer Durchbohrung (4) zum Einsetzen eines Nadelkonus (3) einer Spritze (1) versehen ist und einen auf die Öffnung des Aufnahmegefäßes aufsteckbaren Steckstopfen (36) hat,
**dadurch gekennzeichnet,**
**daß** das aus einer Quarzküvette (5) bestehende Aufnahmegefäß an der dem Adapterstopfen (47; 57) gegenüberliegenden Seite mit einem stopfenartigen Verschlußelement (60) verschlossen ist,
und **daß** eine Lüftungsbohrung (61) seitlich am Steckstopfen des Verschlußelements (60) in einem durch das Darüberschieben der Quarzküvette (5) verschließbaren Bereich angebracht ist.

## Claims

1. Receiving vessel for bodily fluids with an adapter plug (47) which is provided with a closable ventilation bore and which for filling with and emptying of bodily fluids is provided with a through-bore (4) for the insertion of a needle cone (3) of a syringe (1) and has a push-fit plug (36) which can be fitted on the opening of the receiving vessel, **characterised in that** the receiving vessel consisting of a quartz cuvette (5) is closed on the side opposite the adapter plug (47; 57) with a plug-like closure element (29; 21, 39; 31, 49; 45, 59; 41, 46; 60), and **in that** the ventilation bore (42) is formed on the push-fit plug (36) of the adapter plug (47) in a region closable by fitting the quartz cuvette (5) over it.

2. Receiving vessel according to claim 1, **characterised in that** a through-bore (25; 35) of the plug-like closure element (39; 49) is closable with a sealing cap (21; 31).

3. Receiving vessel according to claim 1, **characterised in that** the closure element (41; 59) is provided with a gas-permeable and liquid-impermeable membrane (45; 46).

4. Receiving vessel according to claim 3, **characterised in that** the membrane (46) is held in or with an annular cap (41).

5. Receiving vessel for bodily fluids with an adapter plug (47; 57) which for filling with and emptying of bodily fluids is provided with a through-bore (4) for the insertion of a needle cone (3) of a syringe (1) and has a push-fit plug (36) which can be fitted on the opening of the receiving vessel, **characterised in that** the receiving vessel consisting of a quartz cuvette (5) is closed on the side opposite the adapter plug (47; 57) with a plug-like closure element (60), and **in that** a ventilation bore (61) is formed laterally on the push-fit plug of the closure element (60) in a region closable by fitting the quartz cuvette (5) over it.

## Revendications

1. Récipient pour liquides biologiques, comprenant un bouchon adaptateur (47) pourvu d'un perçage d'aération obturable, qui est pourvu, pour assurer l'introduction et le déchargement de liquides biologiques, d'un perçage traversant (4) permettant d'insérer le manchon conique d'aiguille (3) d'une seringue (1) et possède un bouchon enfichable (36) susceptible d'être enfiché sur l'ouverture du récipient,
**caractérisé en ce que**
le récipient, qui est constitué par une cuvette en quartz (5) est fermé, du côté opposé au bouchon adaptateur (47 ; 57), par un élément de fermeture du type bouchon (29 ; 21, 39 ; 31, 49 ; 45, 59 ; 41, 46 ; 60),
**et en ce que** le perçage d'aération (42) est ménagé sur le bouchon enfichable (36) du bouchon adaptateur (47), dans une zone qui peut être obturée en faisant glisser la cuvette en quartz (5) dessus.

2. Récipient selon la revendication 1,
**caractérisé en ce qu'**un perçage traversant (25 ; 35) de l'élément de fermeture (39 ; 49) du type bouchon est susceptible d'être obturé par un capuchon de fermeture (21 ; 31).

3. Récipient selon la revendication 1,
**caractérisé en ce que** l'élément de fermeture (41 ; 59) est pourvu d'une membrane (45 ; 46) perméable aux gaz et imperméable aux liquides.

4. Récipient selon la revendication 3,
**caractérisé en ce que** la membrane (46) est maintenue dans ou par un capuchon annulaire (41).

5. Récipient pour liquides biologiques, comprenant un bouchon adaptateur (47 ; 57) qui est pourvu, pour assurer l'introduction et le déchargement de liquides biologiques, d'un perçage traversant (4) permettant d'insérer le manchon conique d'aiguille (3) d'une seringue (1) et possède un bouchon enfichable (36) susceptible d'être enfiché sur l'ouverture du récipient,
**caractérisé en ce que**
le récipient, qui est constitué par une cuvette en quartz (5) est fermé, du côté opposé au bouchon adaptateur (47 ; 57), par un élément de fermeture du type bouchon (60),
**et en ce qu'un** perçage d'aération (61) est ménagé sur le côté du bouchon enfichable de l'élément de fermeture (60), dans une zone qui peut être obturée en faisant glisser la cuvette en quartz (5) dessus.
